# EUROPEAN PATENT APPLICATION

(11) **EP 2 998 300 A1**
(43) Date of publication of application: **23.03.2016**
(21) Application number: 13884517.7
(22) Date of filing: 08.08.2013
(51) Int. Cl.: C07D 251/24, C07D 471/04, C09K 11/06, H01L 51/50

(54) **ORGANIC COMPOUND, ORGANIC OPTOELECTRONIC ELEMENT, AND DISPLAY DEVICE**

(30) Priority: 16.05.2013 KR 20130055955
(71) Applicant: Cheil Industries Inc., Gumi-si, Gyeongsangbuk-do 730-710 (KR)
(72) Inventor: LEE, Han-Ill, Uiwang-si Gyeonggi-do 437-711 (KR); YU, Eun-Sun, Uiwang-si Gyeonggi-do 437-711 (KR); KANG, Dong-Min, Uiwang-si Gyeonggi-do 437-711 (KR); KANG, Eui-Su, Uiwang-si Gyeonggi-do 437-711 (KR); PARK, Young-Sung, Uiwang-si Gyeonggi-do 437-711 (KR); SHIN, Ji-Hun, Uiwang-si Gyeonggi-do 437-711 (KR); YANG, Yong-Tak, Uiwang-si Gyeonggi-do 437-711 (KR); OH, Jae-Jin, Uiwang-si Gyeonggi-do 437-711 (KR); RYU, Dong-Kyu, Uiwang-si Gyeonggi-do 437-711 (KR); LEE, Sang-Shin, Uiwang-si Gyeonggi-do 437-711 (KR); JANG, Yu-Na, Uiwang-si Gyeonggi-do 437-711 (KR); JEONG, Soo-Young, Uiwang-si Gyeonggi-do 437-711 (KR); HAN, Su-Jin, Uiwang-si Gyeonggi-do 437-711 (KR); HONG, Jin-Seok, Uiwang-si Gyeonggi-do 437-711 (KR)
(74) Representative: Michalski Hüttermann & Partner Patentanwälte mbB
(86) International application number: PCT/KR2013/007167
(87) International publication number: WO 2014/185589

(57) **Abstract**

An organic compound represented by Chemical Formula 1, an organic optoelectronic device including the organic compound, and a display device including the organic optoelectronic device are disclosed.

## Description

### [Technical Field]

An organic compound, an organic optoelectronic device and a display device are dis.

### [Background Art]

An organic optoelectronic device is a device that converts electrical energy into photoenergy, and vice versa.

An organic optoelectronic device may be classified as follows in accordance with its driving principles. One is an optoelectronic device where excitons are generated by photoenergy, separated into electrons and holes, and are transferred to different electrodes to generate electrical energy, and the other is a light emitting device where a voltage or a current is supplied to an electrode to generate photoenergy from electrical energy.

Examples of the organic optoelectronic device may be an organic photoelectric device, an organic light emitting diode, an organic solar cell, and an organic photo conductor drum.

Of these, an organic light emitting diode OLED has recently drawn attention due to an increase in demand for flat panel displays.

Such an organic light emitting diode converts electrical energy into light by applying current to an organic light emitting material. It has a structure in which an organic layer is interposed between an anode and a cathode. Herein, the organic layer may include an emission layer and optionally an auxiliary layer, and the auxiliary layer may be, for example at least one selected from a hole injection layer, a hole transport layer, an electron blocking layer, an electron transport layer, an electron injection layer, and a hole blocking layer for improving efficiency and stability of an organic light emitting diode.

Performance of an organic light emitting diode may be affected by characteristics of the organic layer, and among them, may be mainly affected by characteristics of an organic material of the organic layer.

Particularly, development for an organic material being capable of increasing hole and electron mobility and simultaneously increasing electrochemical stability is needed so that the organic light emitting diode may be applied to a large-size flat panel display.

### [Disclosure]

### [Technical Problem]

One embodiment provides an organic compound being capable of realizing an organic optoelectronic device having improved driving voltage and efficiency.

Another embodiment provides an organic optoelectronic device including the organic compound.

Yet another embodiment provides a display device including the organic optoelectronic device.

### [Technical Solution]

According to one embodiment, an organic compound represented by Chemical Formula 1 is provided.

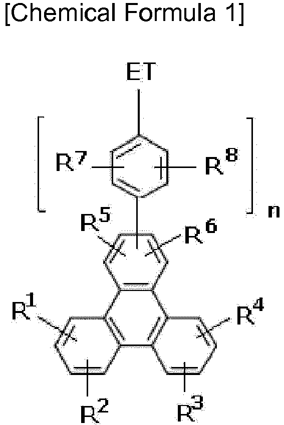

In Chemical Formula 1,
ET is a functional group being capable of accepting an electron when an electric field is applied,
R¹ to R⁶ are independently hydrogen, deuterium, a substituted or unsubstituted C1 to C30 alkyl group, a substituted or unsubstituted C1 to C12 aryl group, or a combination thereof,
R⁷ and R⁸ are independently hydrogen, deuterium, a substituted or unsubstituted C1 to C10 alkyl group or are linked to each other to form a fused ring, and
n is an integer ranging from 0 to 3.

According to another embodiment, an organic optoelectronic device includes an anode and a cathode facing each other, at least one organic layer between the anode and the cathode, wherein the organic layer includes the organic compound.

According to yet another embodiment, a display device including the organic optoelectronic device is provided.

### [Advantageous Effect]

An organic optoelectronic device having high efficiency and long lifespan may be realized.

### [Description of Drawings]

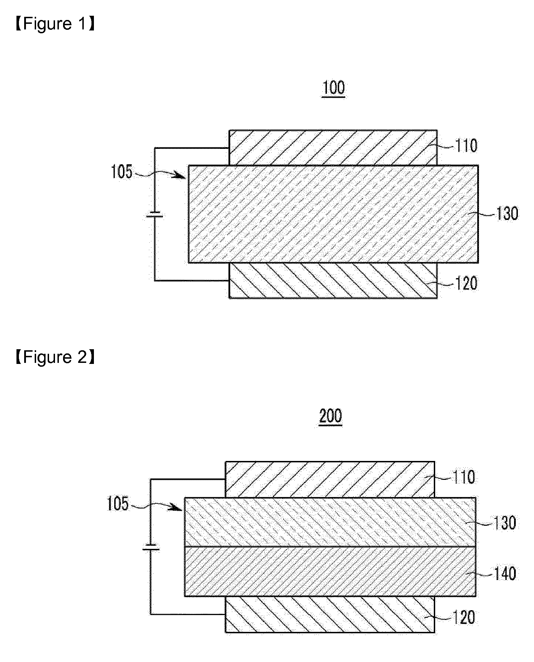

FIGS. 1 and 2 are cross-sectional views showing organic light emitting diodes according to one embodiment.

### [Best Mode]

Hereinafter, embodiments of the present invention are described in detail. However, these embodiments are exemplary, the present invention is not limited thereto and the present invention is defined by the scope of claims.

In the present specification, when a definition is not otherwise provided, the term "substituted" refers to one substituted with a deuterium, a halogen, a hydroxy group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C3 to C30 cycloalkyl group, a C3 to C30 heterocycloalkyl group, a C6 to C30 aryl group, a C6 to C30 heteroaryl group, a C1 to C20 alkoxy group, a fluoro group, a C1 to C10 trifluoroalkyl group such as a trifluoromethyl group, or a cyano group, instead of at least one hydrogen of a substituent or a compound.

In addition, two adjacent substituents of the substituted halogen, hydroxy group, an amino group, a substituted or unsubstituted C1 to C20 amine group, a nitro group, a substituted or unsubstituted C3 to C40 silyl group, C1 to C30 alkyl group, C1 to C10 alkylsilyl group, C3 to C30 cycloalkyl group, C3 to C30 heterocycloalkyl group, C6 to C30 aryl group, C6 to C30 heterocyclic group, C1 to C20 alkoxy group, fluoro group, C1 to C10 trifluoroalkyl group such as trifluoromethyl group and the like, or cyano group may be fused with each other to form a ring. For example, the substituted C6 to C30 aryl group may be fused with another adjacent substituted C6 to C30 aryl group to form a substituted or unsubstituted fluorene ring.

In the present specification, when specific definition is not otherwise provided, "hetero" refers to one including 1 to 3 hetero atoms selected from the group consisting of N, O, S, P, and Si, and remaining carbons in one compound or substituent.

In the present specification, when a definition is not otherwise provided, the term "combination thereof" refers to at least two substituents bound to each other by a linker, or at least two substituents condensed to each other.

In the present specification, when a definition is not otherwise provided, "alkyl group" refers to an aliphatic hydrocarbon group. The alkyl group may be "a saturated alkyl group" without any double bond or triple bond.

The alkyl group may be a C1 to C30 alkyl group. More specifically, the alkyl group may be a C1 to C20 alkyl group or a C1 to C10 alkyl group. For example, a C1 to C4 alkyl group may have 1 to 4 carbon atoms in an alkyl chain which may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group, a cyclopropyl group, a cyclobutyl group, a cyclopentyl group, a cyclohexyl group, and the like.

In the present specification, "aryl group" refers to a cyclic substituent where all elements have p-orbitals, and these p-orbitals forms conjugation, and includes a monocyclic, polycyclic or fused ring polycyclic (i.e., rings sharing adjacent pairs of carbon atoms) functional group.

In the present specification, "heteroaryl group" refers to aryl group including 1 to 3 hetero atoms selected from N, O, S, P and Si, and remaining carbons. When the heteroaryl group is a fused ring, each ring may include 1 to 3 hetero atoms.

More specifically, the substituted or unsubstituted C6 to C30 aryl group and/or the substituted or unsubstituted C2 to C30 heteroaryl group refer to a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted fluorenyl group, a substituted or unsubstituted dibenzofuranyl group, a substituted or unsubstituted dibenzothiophenyl group, a substituted or unsubstituted carbazolyl group, or a combination thereof, but is not limited thereto.

In the present specification, being fused with each other to form a ring refers to forming naphthalene, triphenylene, anthracene, phenanthrene, tetralin, quinoline, indene, indane, and the like.

In the present specification, hole characteristics refer to characteristics to donate an electron and to form a hole when an electric field is applied, and characteristics that holes formed in the anode is easily injected into the emission layer and transported in the emission layer due to conductive characteristics according to HOMO level.

Electron characteristics refer to characteristics to accept an electron when an electric field is applied, and characteristics that electron formed in the cathode is easily injected into the emission layer and transported in the emission layer due to conductive characteristics according to LUMO level.

Hereinafter, an organic compound according to one embodiment is described.

An organic compound according to one embodiment is represented by Chemical Formula 1.

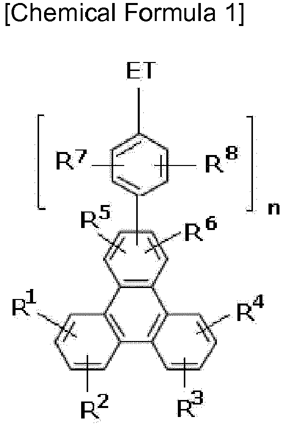

In Chemical Formula 1,
ET is a functional group being capable of accepting an electron when an electric field is applied,
R¹ to R⁶ are independently hydrogen, deuterium, a substituted or unsubstituted C1 to C30 alkyl group, a substituted or unsubstituted C1 to C12 aryl group, or a combination thereof,
R⁷ and R⁸ are independently hydrogen, deuterium, a substituted or unsubstituted C1 to C10 alkyl group or are linked to each other to form a fused ring, and
n is an integer ranging from 0 to 3.

The organic compound may be, for example represented by Chemical Formula 1-I or Chemical Formula 1-II depending on a bonding position of a triphenylene group.

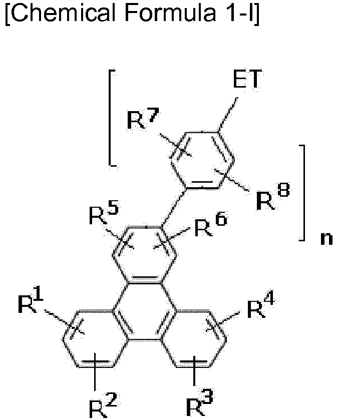

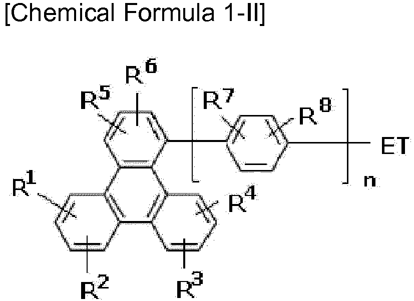

In Chemical Formula 1-I or 1-II, R¹ to R⁸, ET and n are the same as described above.

The organic compound represented by Chemical Formula 1 includes a triphenylene group and a substituent having electron characteristics.

The organic compound includes a substituent having electron characteristics and thus, has a structure of easily accepting electrons when an electric field is applied and resultantly, may decrease a driving voltage of an organic optoelectronic device when applied thereto.

In addition, the organic compound includes a triphenylene moiety easily accepting holes and a moiety easily accepting electrons and thus, has a bipolar structure and balances between hole and electron flows and resultantly, may improve efficiency of an organic optoelectronic device when applied thereto.

In addition, the organic compound appropriately may localize the triphenylene moiety easily accepting holes and the moiety easily accepting electrons and control a flow of a conjugated system and thus, show excellent bipolar characteristics. Accordingly, the organic compound may improve lifespan of an organic optoelectronic device.

In addition, the organic compound has a structure of being able to effectively prevent stacking of the organic compounds and thus, may deteriorate process stability and simultaneously, lower a deposit temperature.

The ET may include, for example a heteroaryl group including at least one nitrogen.

The ET may be, for example a functional group represented by Chemical Formula 2.

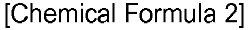

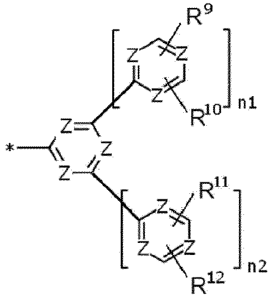

In Chemical Formula 2,
Z is each independently N or CR^{a},
at least one of Z is N,
R⁹ to R¹² and R^{a} are each independently hydrogen, deuterium, a substituted or unsubstituted C1 to C10 alkyl group, a substituted or unsubstituted C6 to C12 aryl group, or a combination thereof, and
n1 and n2 are each independently 0 or 1.

The functional group represented by Chemical Formula 2 may be, for example one of functional groups listed in Group 1.

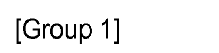

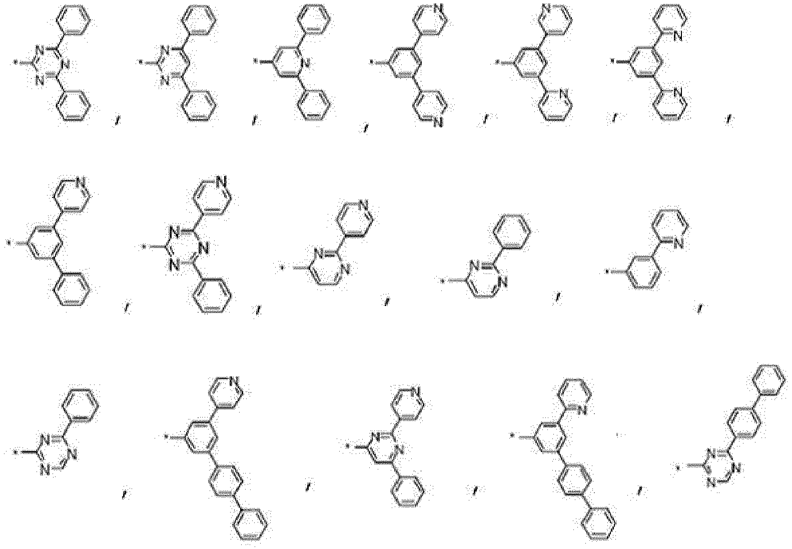

The ET may be, for example a substituted or unsubstituted functional group listed in Group 2.

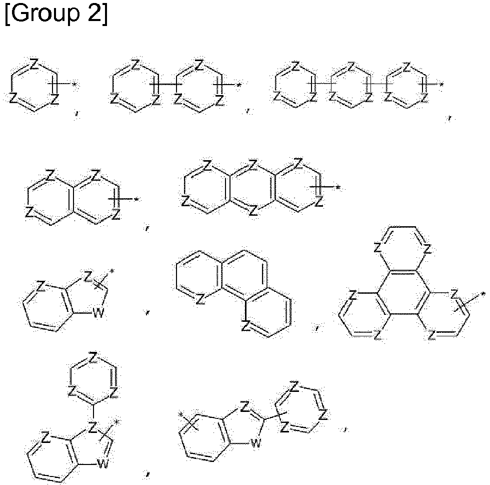

In Group 2,
W is each independently N, O, S, SO, SO₂, CR^{c}, CR^{d}R^{e}, SiR^{f} or SiR^{g}R^{h},
Z is each independently N, C or CRⁱ,
wherein R^{c} to Rⁱ are each independently hydrogen, deuterium, a substituted or unsubstituted C1 to C30 alkyl group, a substituted or unsubstituted C3 to C30 cycloalkyl group, a substituted or unsubstituted C3 to C30 heterocycloalkyl group, a substituted or unsubstituted C6 to C30 aryl group, a substituted or unsubstituted C3 to C30 heteroaryl group, a substituted or unsubstituted amine group, a substituted or unsubstituted C6 to C30 arylamine group, a substituted or unsubstituted C6 to C30 heteroarylamine group, a substituted or unsubstituted C1 to C30 alkoxy group, a substituted or unsubstituted C2 to C30 alkoxycarbonyl group, a substituted or unsubstituted C2 to C30 alkoxycarbonylamino group, a substituted or unsubstituted C7 to C30 aryloxycarbonylamino group, a substituted or unsubstituted C1 to C30 sulfamoylamino group, a substituted or unsubstituted C2 to C30 alkenyl group, a substituted or unsubstituted C2 to C30 alkynyl group, a substituted or unsubstituted silyl group, a substituted or unsubstituted silyloxy group, a substituted or unsubstituted C1 to C30 acyl group, a substituted or unsubstituted C1 to C20 acyloxy group, a substituted or unsubstituted C1 to C20 acylamino group, a substituted or unsubstituted C1 to C30 sulfonyl group, a substituted or unsubstituted C1 to C30 alkylthiol group, a substituted or unsubstituted C1 to C30 heterocyclothiol group, a substituted or unsubstituted C6 to C30 arylthiol group, a substituted or unsubstituted C1 to C30 heteroarylthiol group, a substituted or unsubstituted C1 to C30 ureide group, a halogen, a halogen-containing group, a cyano group, a hydroxyl group, an amino group, a nitro group, a carboxyl group, a ferrocenyl group, or a combination thereof, and
* is a linking point at which an element of the functional group is linked.

The substituted or unsubstituted functional group listed in Group 2 may be, for example a substituted or unsubstituted functional group listed in Group 3.

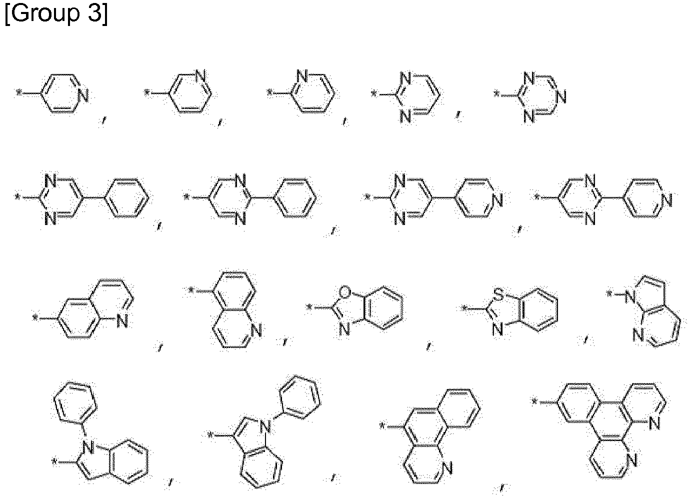

The organic compound may be, for example compounds listed in Group 4, but is not limited thereto.

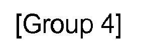

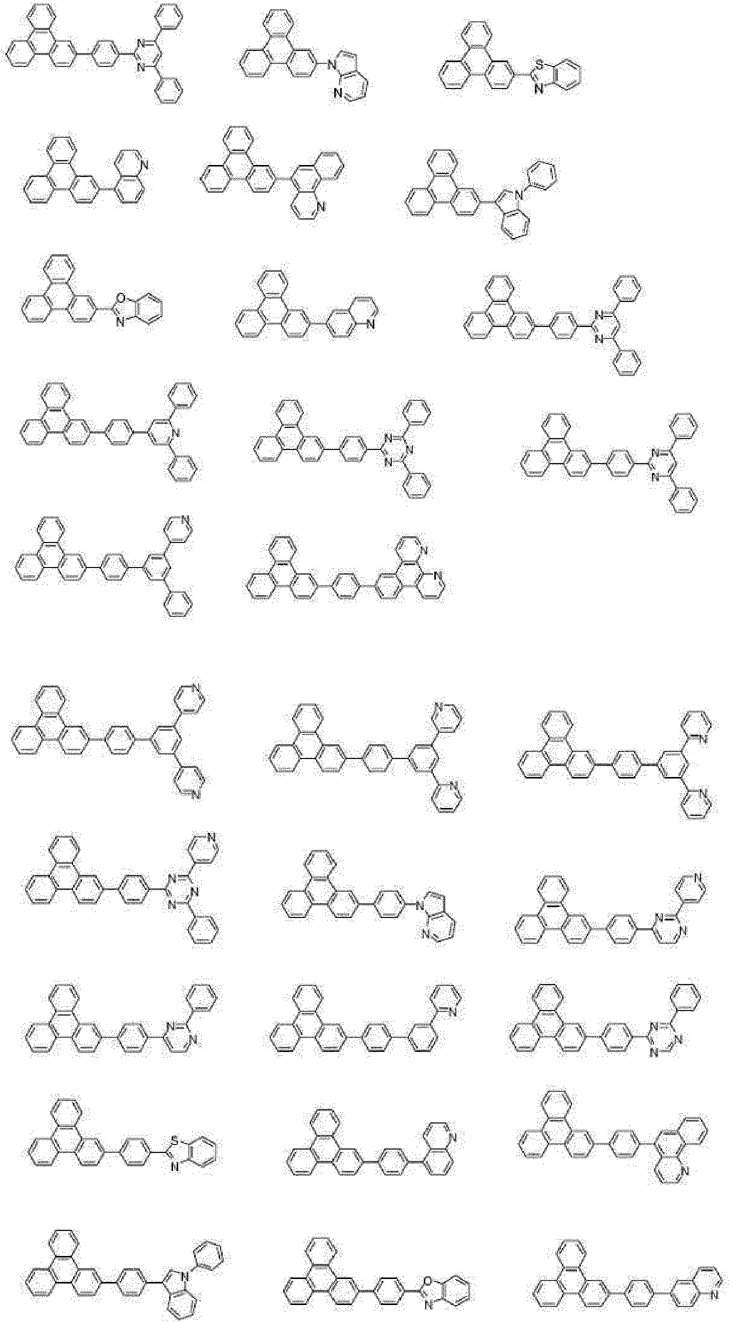

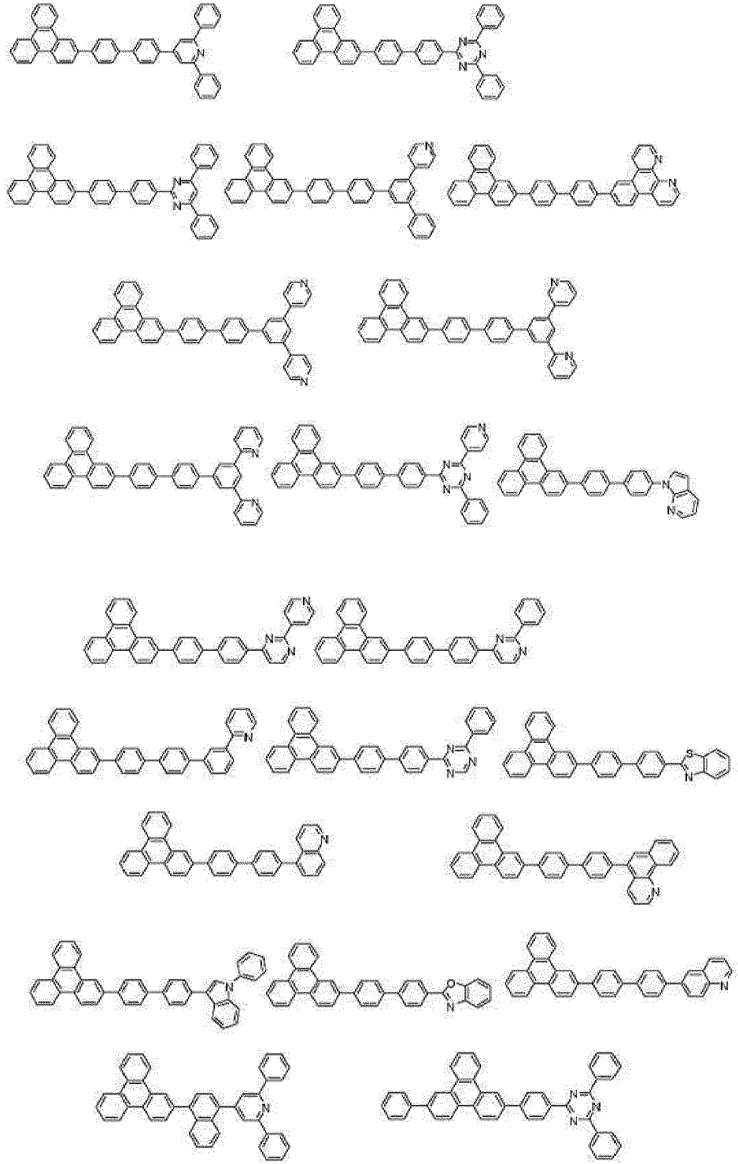

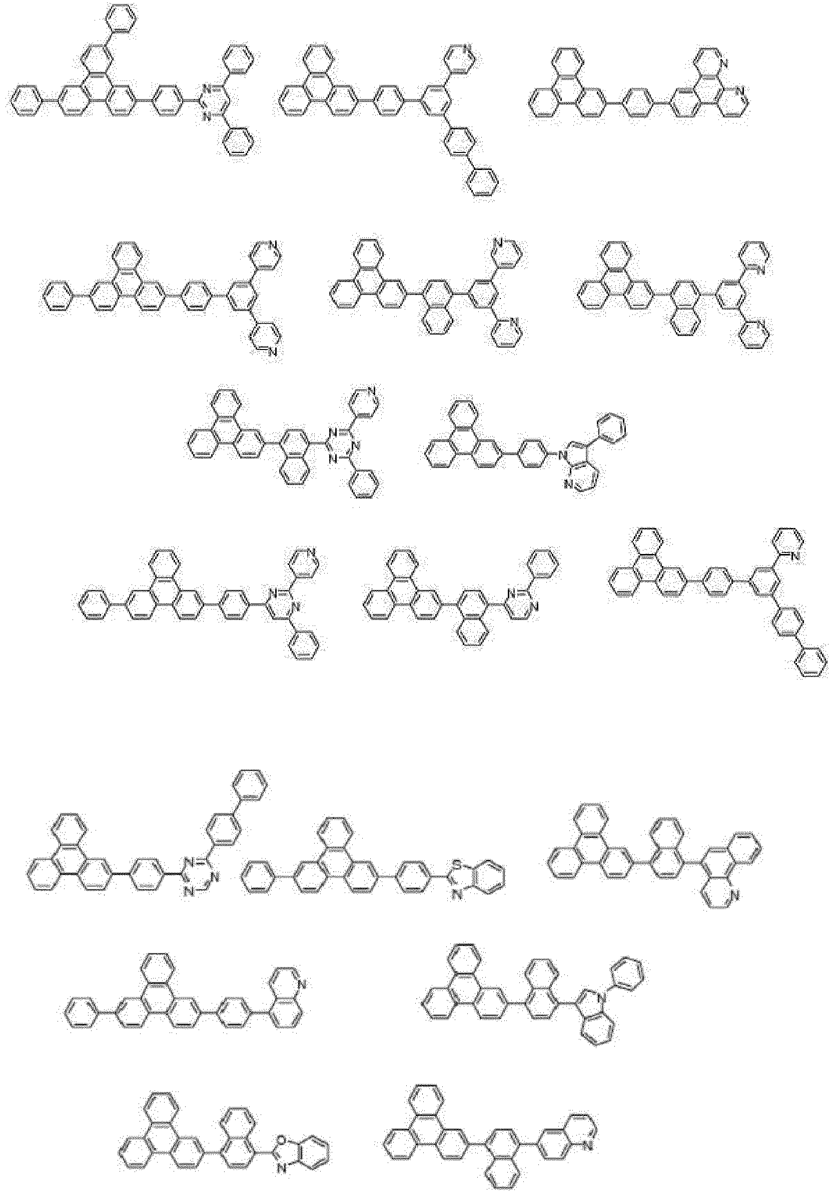

The organic compound may have LUMO energy of about -2.0 to -2.5 eV. Electron injection characteristics may increase within the LUMO energy range.

Hereinafter, an organic optoelectronic device including the organic compound is described.

The organic optoelectronic device may be any device to convert electrical energy into photoenergy and vice versa without particular limitation, and may be, for example an organic photoelectric device, an organic light emitting diode, an organic solar cell, and an organic photo-conductor drum.

Herein, an organic light emitting diode as one example of an organic optoelectronic device is described.

FIGS. 1 and 2 are cross-sectional views of an organic light emitting diode according to one embodiment.

Referring to FIG. 1, an organic optoelectronic device 100 according to one embodiment includes an anode 120 and a cathode 110 facing each other and an organic layer 105 between the anode 120 and the cathode 110.

The anode 120 may be made of a conductor having a high work function to help hole injection, for example metal, a metal oxide and/or a conductive polymer. The anode 120 may include, for example a metal or an alloy thereof such as nickel, platinum, vanadium, chromium, copper, zinc, and gold; metal oxide such as zinc oxide, indium oxide, indium tin oxide (ITO), and indium zinc oxide (IZO); a combination of a metal and an oxide such as ZnO and Al or SnO₂ and Sb; a conductive polymer such as poly (3-methylthiophene), poly (3,4-(ethylene-1,2-dioxy)thiophene) (PEDT), polypyrrole and polyaniline, but is not limited thereto.

The cathode 110 may be made of a conductor having a low work function to help electron injection, for example a metal, a metal oxide and/or a conductive polymer. The cathode 110 may include, for example a metal or an alloy thereof such as magnesium, calcium, sodium, potassium, titanium, indium, yttrium, lithium, gadolinium, aluminum silver, tin, lead, cesium, barium, and the like; a multi-layer structured material such as LiF/AI, LiO₂/Al, LiF/Ca, LiF/Al and BaF₂/Ca, but is not limited thereto.

The organic layer 105 includes an emission layer 130 including the organic compound.

The emission layer 130 may include, for example the organic compound at alone or with at least two of the organic compounds, or as a mixture with other different compound from the organic compound. When the organic compound is mixed with the other compound, for example they may be included as a host and a dopant, wherein the organic compound may be, for example included as a host. The host may be, for example phosphorescent host or fluorescent host, for example a phosphorescent host.

When the organic compound is included as a host, the dopant may be selected from well-known inorganic, organic, organic/inorganic compound as a dopant.

Referring to FIG. 2, an organic light emitting diode 200 further includes a hole auxiliary layer 140 as well as an emission layer 230. The hole auxiliary layer 140 may further increase hole injection and/or hole transport between the anode 120 and emission layer 230 and block electrons. The hole auxiliary layer 140 may be, for example a hole transport layer HTL, a hole injection layer HIL, and/or an electron blocking layer, and may include at least one layer. The organic compound may be included in the emission layer 130 and/or the hole auxiliary layer 140.

In one embodiment of the present invention, in FIG. 1 or 2, the organic thin layer 105 may further include an auxiliary electron transport layer ETL, an electron transport layer ETL, an electron injection layer EIL, an auxiliary hole transport layer HTL, a hole injection layer HIL, and the like. The organic compound may be included in an emission layer 130 and/or a hole auxiliary layer 140, or may be included in an auxiliary electron transport layer ETL, an electron transport layer ETL, an electron injection layer EIL, an auxiliary hole transport layer HTL and/or a hole injection layer HIL.

The organic light emitting diodes 100 and 200 may be manufactured by forming an anode or a cathode on a substrate, forming an organic layer in accordance with a dry coating method such as evaporation, sputtering, plasma plating, and ion plating; or a wet coating method such as spin coating, slit coating, dipping, flow coating and inkjet printing; and forming a cathode or an anode thereon.

The organic light emitting diode may be applied to an organic light emitting diode OLED display.

### [Mode for Invention]

Hereinafter, the embodiments are illustrated in more detail with reference to examples. These examples, however, are not in any sense to be interpreted as limiting the scope of the invention.

### Synthesis of Organic Compound

### Representative Synthesis Method

A representative synthesis method is expressed as the following representative Reaction Scheme.

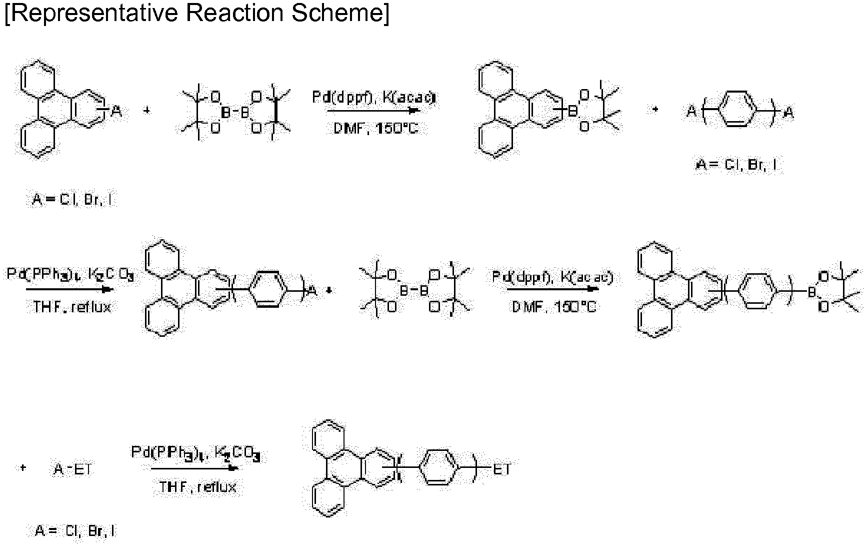

### Synthesis Example 1: Synthesis of Intermediate I-1

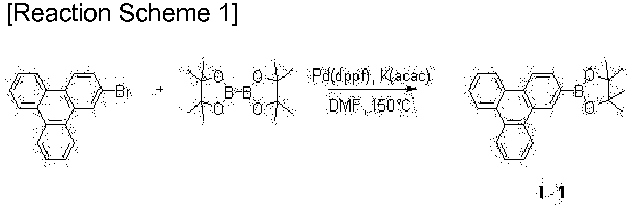

100 g (326 mmol) of 2-bromotriphenylene was dissolved in 1L of dimethylforamide (DMF) in a nitrogen environment, 99.2 g (391 mmol) of bis(pinacolato)diboron, 2.66 g (3.26 mmol) of (1,1'-bis(diphenylphosphine)ferrocene)dichloropalladium (II) and 80 g (815 mmol) of potassium acetate were added thereto, and the mixture was heated and refluxed at 150 °C for 5 hours. When the reaction was complete, water was added to the reaction solution, and the mixture was filtered and then, dried in a vacuum oven. The obtained residue was separated through flash column chromatography, obtaining a compound **I-1** (113 g, 98 %).
HRMS (70 eV, EI+): m/z calcd for C24H23BO2: 354.1791, found: 354.
Elemental Analysis: C, 81 %; H, 7 %

### Synthesis Example 2: Synthesis of Intermediate I-2

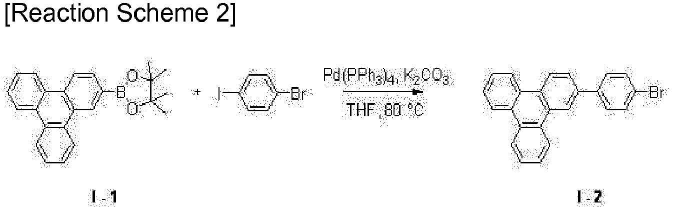

100 g (282 mmol) of the compound **I-1** was dissolved in 1 L of tetrahydrofuran (THF) in a nitrogen environment, 95.9 g 9339 mmol) of 1-bromo-4-iodobenzene and 3.26 g (2.82 mmol) of tetrakis(triphenylphosphine)palladium were added thereto, and the mixture was agitated. Then, 97.4 g (705 mmol) of potassium carbonate saturated in water was added thereto, and the resulting mixture was heated and refluxed at 80 °C for 16 hours. When the reaction was complete, water was added to the reaction solution, and then, a product obtained after using dichloromethane (DCM) to perform extraction and anhydrous MgSO4 to remove moisture was filtered and concentrated under a reduced pressure. The obtained residue was separated and purified through flash column chromatography, obtaining a compound I-2 (98.4 g, 91 %).
HRMS (70 eV, EI+): m/z calcd for C24H15Br: 382.0357, found: 382.
Elemental Analysis: C, 75 %; H, 4 %

### Synthesis Example 3: Synthesis of Intermediate I-3

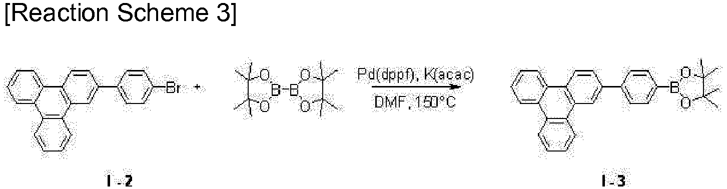

90 g (235 mmol) of the compound I-2 was dissolved in 0.8 L of dimethylforamide (DMF) in a nitrogen environment, 71.6 g (282 mmol) of bis(pinacolato)diboron, 1.92 g (2.35 mmol) of (1,1'-bis(diphenylphosphine)ferrocene)dichloropalladium (II) and 57.7 g (588 mmol) of potassium acetate were added thereto, and the mixture was heated and refluxed at 150 °C for 26 hours. When the reaction was complete, water was added to the reaction solution, and the mixture was filtered and then, dried in a vacuum oven. The obtained residue was separated and purified through flash column chromatography, obtaining a compound I-3 (86.0 g, 85 %).
HRMS (70 eV, EI+): m/z calcd for C30H27BO2: 430.2104, found: 430.
Elemental Analysis: C, 84 %; H, 6 %

### Synthesis Example 4: Synthesis of Intermediate I-4

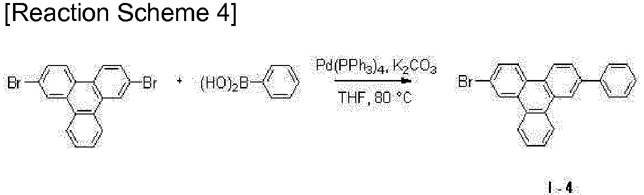

100 g (259 mmol) of 2,7-dibromotriphenylene was dissolved in 0.7 L of tetrahydrofuran (THF) in a nitrogen environment, 31.6 g (259 mmol) of phenylboronic acid and 2.99 g (2.59 mmol) of tetrakis(triphenylphosphine)palladium were added thereto, and the mixture was agitated. Then, 89.5 g (648 mmol) of potassium carbonate saturated in water was added thereto, and the mixture was heated and refluxed at 80 °C for 7 hours. When the reaction was compete, water was added to the reaction solution, and a product obtained after performing extraction with dichloromethane (DCM) and removing moisture with anhydrous MgSO4 was filtered and concentrated under a reduced pressure. The obtained residue was separated and purified through flash column chromatography, obtaining a compound I-4 (79.4 g, 80 %).
HRMS (70 eV, EI+): m/z calcd for C24H15Br: 382.0357, found: 382.
Elemental Analysis: C, 75 %; H, 4 %

### Synthesis Example 5: Synthesis of Intermediate I-5

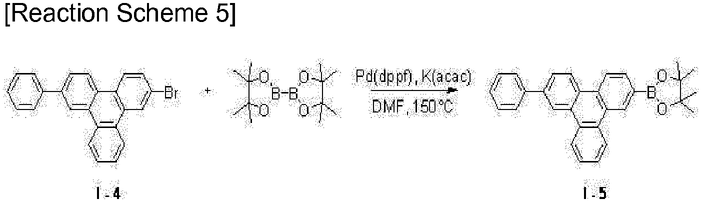

70 g (183 mmol) of the compound I-4 was dissolved in 1L of dimethylforamide (DMF) in a nitrogen environment, 55.7 g (219 mmol) of bis(pinacolato)diboron and 1.49 g (1.83 mmol) of (1,1'-bis(diphenylphosphine)ferrocene)dichloropalladium (II) and 44.9 g (458 mmol) of potassium acetate were added thereto, and the mixture was heated and refluxed at 150 °C for 7 hours. When the reaction was complete, water was added to the reaction solution, and the mixture was filtered and then, dried in a vacuum oven. The obtained residue was separated and purified through flash column chromatography, obtaining a compound I-5 (54.3 g, 69 %).
HRMS (70 eV, EI+): m/z calcd for C30H27BO2: 430.2104, found: 430.
Elemental Analysis: C, 84 %; H, 6 %

### Synthesis Example 6: Synthesis of Intermediate I-6

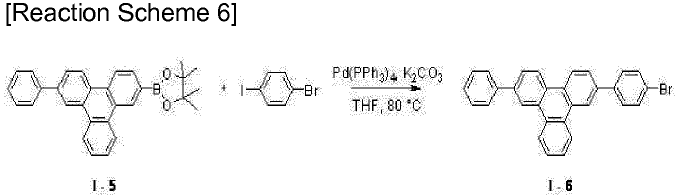

50 g (116 mmol) of the compound I-5 was dissolved in 0.4 L of tetrahydrofuran (THF) in a nitrogen environment, 32.9 g (116 mmol) of 1-bromo-4-iodobenzene and 1.34 g (1.16 mmol) of tetrakis(triphenylphosphine)palladium were added thereto, and the mixture was agitated. Then, 40.1 g (290 mmol) of potassium carbonate saturated in water was added thereto, and the resulting mixture was heated and refluxed at 80 °C for 12 hours. When the reaction was complete, water was added to the reaction solution, and a product obtained after performing extraction with dichloromethane (DCM) and removing moisture with anhydrous MgSO4 was filtered and concentrated under a reduced pressure. The obtained residue was separated and purified through flash column chromatography, obtaining a compound I-6 (53.3 g, 81 %).
HRMS (70 eV, EI+): m/z calcd for C30H19Br: 458.0670, found: 458.
Elemental Analysis: C, 78 %; H, 4 %

### Synthesis Example 7: Synthesis of Intermediate I-7

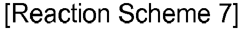

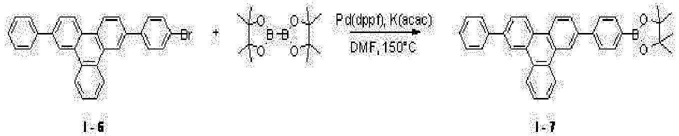

50 g (109 mmol) of the compound I-6 was dissolved in 0.4 L of dimethylforamide (DMF) in a nitrogen environment, 33.2 g (131 mmol) of bis(pinacolato)diboron, 0.89 g (1.09 mmol) of (1,1 - bis(diphenylphosphine)ferrocene)dichloropalladium (II) and 26.7 g (273 mmol) of potassium acetate were added thereto, and the mixture was heated and refluxed at 150 °C for 10 hours. When the reaction was complete, water was added to the reaction solution, and the mixture was filtered and then, died in a vacuum oven. The obtained residue was separated and purified through flash column chromatography, obtaining a compound I-7 (38.6g, 70 %).
HRMS (70 eV, EI+): m/z calcd for C36H31BO2: 506.2417, found: 506.
Elemental Analysis: C, 85 %; H, 6 %

### Synthesis of Final Compound

### Synthesis Example 8: Synthesis of Compound 1

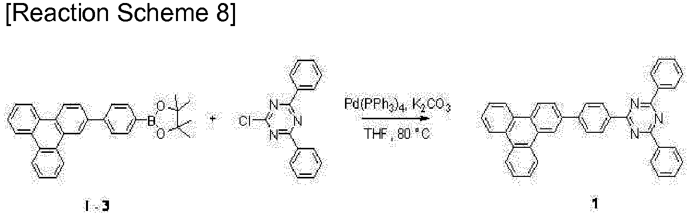

20 g (46.5 mmol) of the compound 1-3 was dissolved in 0.2 L of tetrahydrofuran (THF) in a nitrogen environment, 12.4 g (46.5 mmol) of 2-chloro-4,6-diphenyl-1,3,5-triazine and 0.54 g (0.47 mmol) of tetrakis(triphenylphosphine)palladium were added thereto, and the mixture was agitated. Then, 16.1 g 9116 mmol) of potassium carbonate saturated in water was added thereto, and the mixture was heated and refluxed at 80 °C for 13 hours. When the reaction was complete, water was added to the reaction solution, and a product obtained after extracting the mixture with dichloromethane (DCM) and removing moisture with anhydrous MgSO4 was filtered and then, concentrated under a reduced pressure. The obtained residue was separated and purified through flash column chromatography, obtaining a compound 1 (20.2 g, 81 %).
HRMS (70 eV, EI+): m/z calcd for C39H25N3: 535.2048, found: 535.
Elemental Analysis: C, 87 %; H, 5 %

### Synthesis Example 9: Preparation of Compound 2

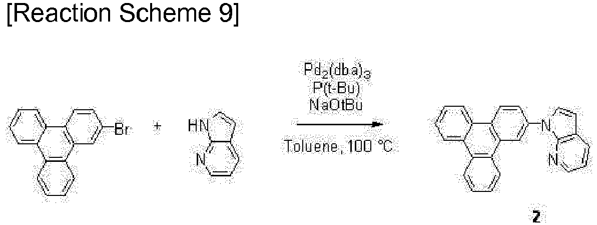

20 g (65.1 mmol) of 2-bromotriphenylene was dissolved in 0.1 L of toluene in a nitrogen environment, 7.7 g (65.1 mmol) of intermediate 1H-pyrrolopyridine, 0.6 g (0.65 mmol) of tris(diphenylideneacetone)dipalladium (o), 0.66 g (3.25 mmol) of tris-tert butylphosphine and 7.51 g (78.1 mmol) of sodium tert-butoxide were sequentially added thereto, and the mixture was heated and refluxed at 100 °C for 16 hours. When the reaction was complete, water was added to the reaction solution, and a product obtained after extracting the mixture with dichloromethane (DCM) and removing moisture with anhydrous MgSO4 was filtered and concentrated under a reduced pressure. The obtained residue was separated and purified through flash column chromatography, obtaining a compound 2 (20.9 g, 93 %).
HRMS (70 eV, EI+): m/z calcd for C25H16N2: 344.1313, found: 344.
Elemental Analysis: C, 87 %; H, 5 %

### Synthesis Example 10: Synthesis of Compound 3

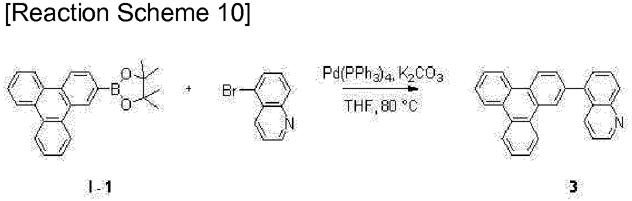

20 g (56.5 mmol) of the compound 1-1 was dissolved in 0.2 L of tetrahydrofuran (THF) in a nitrogen environment, 11.7 g (56.5 mmol) of 5-bromoquinoline and 0.65 g (0.57 mmol) of tetrakis(triphenylphosphine)palladium were added thereto, and the mixture was agitated. Then, 19.5 g (141 mmol) of potassium carbonate saturated in water was added thereto, and the resulting mixture was heated and refluxed at 80 °C for 18 hours. When the reaction was complete, water was added to the reaction solution, and a product obtained after extracting the mixture with dichloromethane (DCM) and removing moisture with anhydrous MgSO4 was filtered and then, concentrated under a reduced pressure. The obtained residue was separated and purified through flash column chromatography, obtaining a compound 3 (18.1 g, 90 %).
HRMS (70 eV, EI+): m/z calcd for C27H17N: 355.1361, found: 355.
Elemental Analysis: C, 91 %; H, 5 %

### Synthesis Example 11: Synthesis of Compound 4

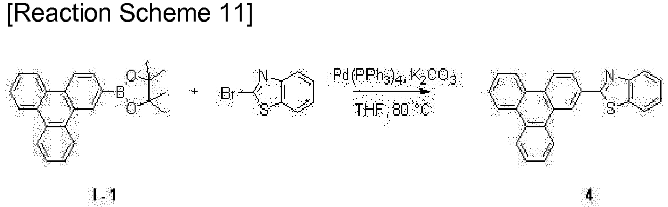

20 g (56.5 mmol) of the compound 1-1 was dissolved in 0.2 L of tetrahydrofuran (THF) in a nitrogen environment, 14.1 g (56.5 mmol) of 2-bromobenzothiazole and 0.65 g (0.57 mmol) of tetrakis(triphenylphosphine)palladium were added thereto, and the mixture was agitated. Then, 19.5 g (141 mmol) of potassium carbonate saturated in water was added thereto, and the mixture was heated and refluxed at 80 °C for 10 hours. When the reaction was complete, water was added to the reaction solution, and a product obtained after extracting the mixture with dichloromethane (DCM) and removing moisture with anhydrous MgSO4 was filtered and concentrated under a reduced pressure. The obtained residue was separated and purified through flash column chromatography, obtaining a compound 4 (17.6 g, 86 %).
HRMS (70 eV, EI+): m/z calcd for C25H15NS: 361.0925, found: 361.
Elemental Analysis: C, 83 %; H, 4 %

### Synthesis Example 12: Synthesis of Compound 5

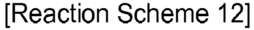

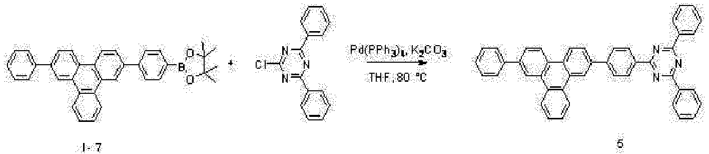

20 g (39.5 mmol) of the compound 1-7 was dissolved in 0.2 L of tetrahydrofuran (THF) in a nitrogen environment, 10.6 g (39.5 mmol) of 2-chloro-4,6-diphenyl-1,3,5-triazine and 0.46 g (0.4 mmol) of tetrakis(triphenylphosphine)palladium were added thereto, and the mixture was agitated. Then, 13.6 g (98.8 mmol) of potassium carbonate saturated in water was added thereto, and the resulting mixture was heated and refluxed at 80 °C for 12 hours. When the reaction was complete, water was added to the reaction solution, and a product obtained after extracting the mixture with dichloromethane (DCM) and removing moisture with anhydrous MgSO4 was filtered and then, concentrated under a reduced pressure. The obtained residue was separated and purified through flash column chromatography, obtaining a compound 5 (20.2 g, 81 %).
HRMS (70 eV, EI+): m/z calcd for C45H29N3: 611.2361, found: 611.
Elemental Analysis: C, 88 %; H, 5 %

### Manufacture of Organic Light Emitting Diode

### Example 1

The compound 1 according to Synthesis Example 8 as a host and Ir(PPy)3 as a dopant were used to manufacture an organic light emitting diode.

As for an anode, 1000 A-thick ITO was used, and as for a cathode, 1000 A-thick aluminum (Al) was used. Specifically, illustrating a method of manufacturing an organic light emitting diode, the anode is manufactured by cutting an ITO glass substrate having 15 Ω/cm² of sheet resistance into a size of 50 mm × 50 mm × 0.7 mm, ultrasonic wave-cleaning them in acetone, isopropylalcohol, and pure water for 5 minutes respectively, and UV ozone cleaning them for 30 minutes. On the substrate, an 800 A-thick hole transport layer (HTL) was formed by depositing N4,N4'-di(naphthalen-1-yl)-N4,N4'-diphenylbiphenyl-4,4'-diamine (NPB) (80 nm) to a vacuum degree of 650×10⁻⁷Pa at a deposit speed raging from 0.1 to 0.3 nm/s. Subsequently, a 300 A-thick emission layer was formed by using the compound 1 according to Synthesis Example 8 under the same vacuum deposit condition, and herein, Ir(PPy)3 as a phosphorescent dopant was simultaneously deposited. Herein, the phosphorescent dopant in an amount of 7 wt% was deposited based on 100 wt% of the emission layer by adjusting the deposit speed of the phosphorescent dopant. On the emission layer, a 50 A-thick hole-blocking layer was formed by depositing bis(2-methyl-8-quinolinolate)-4-(phenylphenolato)aluminum (BAlq) under the same vacuum deposit condition. Subsequently, a 200 A-thick electron transport layer (ETL) was formed by depositing Alq3 under the same vacuum deposit condition. On the electron transport layer (ETL), a cathode was formed by sequentially depositing LiF and Al, manufacturing an organic photoelectric device. The organic photoelectric device had a structure of ITO/ NPB (80 nm)/ EML (compound1 (93 wt%) + Ir(PPy)3 (7 wt%), 30 nm)/ Balq (5 nm)/ Alq3 (20 nm)/ LiF (1 nm) / Al (100 nm).

### Example 2

An organic light emitting diode was manufactured according to the same method as Example 1 except for using the compound 2 according to Synthesis Example 9 instead of the compound 1 according to Synthesis Example 8.

### Example 3

An organic light emitting diode was manufactured according to the same method as Example 1 except for using the compound 3 according to Synthesis Example 10 instead of the compound 1 according to Synthesis Example 8.

### Example 4

An organic light emitting diode was manufactured according to the same method as Example 1 except for using the compound 4 according to Synthesis Example 11 instead of the compound 1 according to Synthesis Example 8.

### Example 5

An organic light emitting diode was manufactured according to the same method as Example 1 except for using the compound 5 according to Synthesis Example 12 instead of the compound 1 according to Synthesis Example 8.

### Comparative Example 1

An organic light emitting diode was manufactured according to the same method as Example 1 except for using CBP instead of the compound 1 according to Synthesis Example 8. The structure of the CBP is provided as follows.

The structures of NPB, BAlq, CBP and Ir(PPy)3 used to manufacture an organic light emitting diode are also provided as follows.

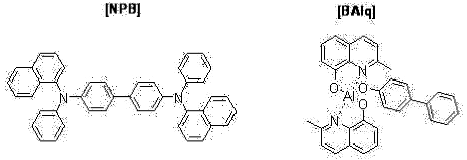

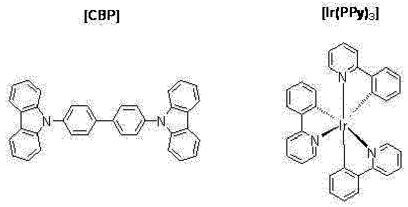

### Evaluation

Current density and luminance changes depending on a voltage and luminous efficiency of each organic light emitting diode according to Example 1 to 5 and Comparative Example 1 were measured. The measurements were specifically performed in the following method, and the results were provided in the following Table 1.

### (1) Measurement of Current Density Change Depending on Voltage Change

Current values flowing in the organic light emitting diodes were measured for, while increasing the voltage using a current-voltage meter (Keithley 2400), and the measured current values were divided by an area to provide the results.

### (2) Measurement of Luminance Change Depending on Voltage Change

Luminance of the organic light emitting diodes was measured for luminance, while increasing the voltage using a luminance meter (Minolta Cs-1000A).

### (3) Measurement of Luminous Efficiency

Current efficiency (cd/A) at the same current density (10 mA/cm2) were calculated by using the luminance, current density, and voltages (V) from the items (1) and (2).

**(Table 1)**

| Devices | Driving voltage (V) | Efficiency (cd/A) | Color (EL color) |
|---|---|---|---|
| Example 1 | 4.1 | 77.0 | Green |
| Example 2 | 3.8 | 84.2 | Green |
| Example 3 | 3.9 | 87.5 | Green |
| Example 4 | 3.4 | 90.0 | Green |
| Example 5 | 4.3 | 65.5 | Green |
| Comparative Example 1 | 4.8 | 31.4 | Green |

Referring to the results of Table 1, the organic light emitting diodes according to Examples 1 to 5 showed a reduced driving voltage and improved luminous efficiency compared with the organic light emitting diode according to Comparative Example 1.

The reason is that the compound for the emission layers used in the organic light emitting diodes according to Examples 1 to 5 smoothed electron and hole flows due to appropriate balance between a triphenylene group well-accepting holes and a substituent well-accepting electrons. Accordingly, the organic light emitting diodes according to Examples 1 to 5 emitted light at a lower driving voltage than the organic light emitting diode according to Comparative Example 1 and simultaneously, showed improved luminous efficiency.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims. Therefore, the aforementioned embodiments should be understood to be exemplary but not limiting the present invention in any way.

## Claims

1. An organic compound represented by the following Chemical Formula 1: 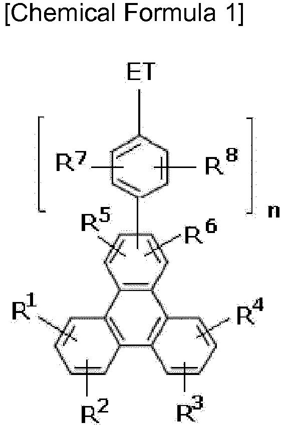 wherein, in Chemical Formula 1,
ET is a functional group being capable of accepting an electron when an electric field is applied,
R¹ to R⁶ are independently hydrogen, deuterium, a substituted or unsubstituted C1 to C30 alkyl group, a substituted or unsubstituted C1 to C12 aryl group, or a combination thereof,
R⁷ and R⁸ are independently hydrogen, deuterium, a substituted or unsubstituted C1 to C10 alkyl group or are linked to each other to form a fused ring, and
n is an integer ranging from 0 to 3.

2. The organic compound of claim 1, wherein the organic compound is represented by Chemical Formula 1-**I** or Chemical Formula 1-II: 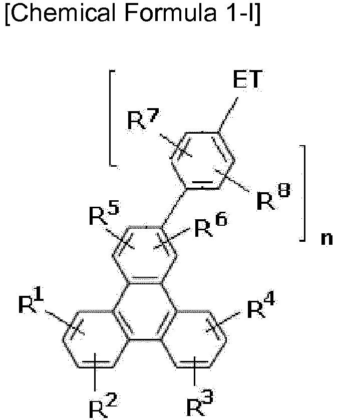 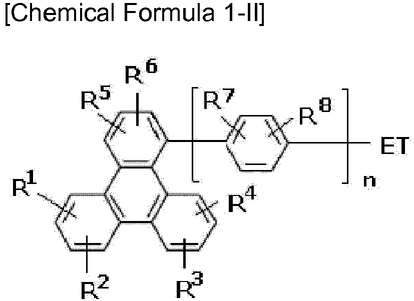 wherein, in Chemical Formula 1-I or 1-II,
ET is a functional group being capable of accepting an electron when an electric field is applied,
R¹ to R⁶ are independently hydrogen, deuterium, a substituted or unsubstituted C1 to C30 alkyl group, a substituted or unsubstituted C1 to C12 aryl group, or a combination thereof,
R⁷ and R⁸ are independently hydrogen, deuterium, a substituted or unsubstituted C1 to C10 alkyl group or are linked to each other to form a fused ring, and
n is an integer ranging from 0 to 3.

3. The organic compound of claim 1, wherein the ET includes a heteroaryl group including at least one nitrogen.

4. The organic compound of claim 3, wherein the ET is a functional group represented by Chemical Formula 2: 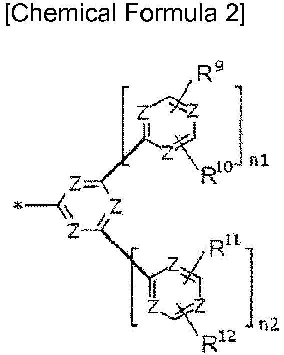 wherein, in Chemical Formula 2,
Z is each independently N or CR^{a},
at least one of Z is N,
R⁹ to R¹² and R^{a} are each independently hydrogen, deuterium, a substituted or unsubstituted C1 to C10 alkyl group, a substituted or unsubstituted C6 to C12 aryl group, or a combination thereof, and
n1 and n2 are each independently 0 or 1.

5. The organic compound of claim 4, wherein the functional group represented by Chemical Formula 2 is one of functional groups listed in Group 1:

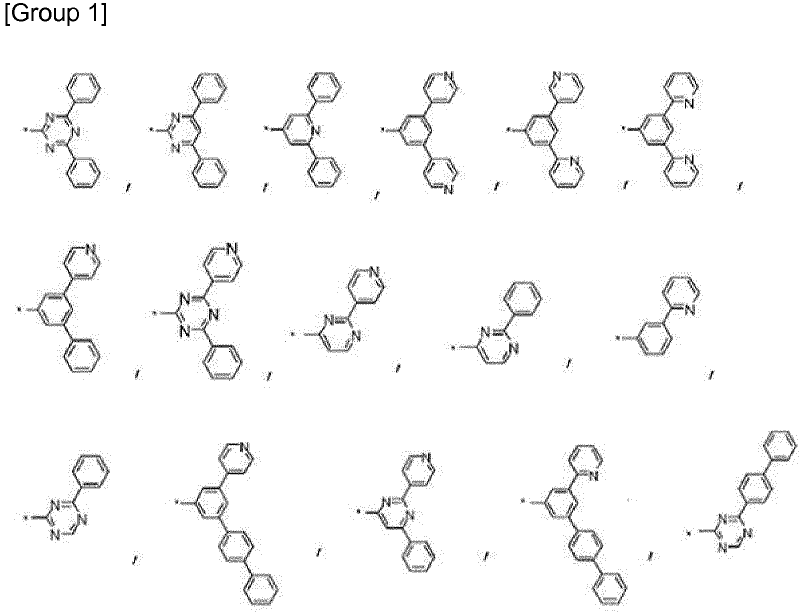

6. The organic compound of claim 1, wherein ET is a substituted or unsubstituted functional group listed in Group 2: 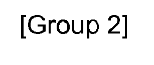 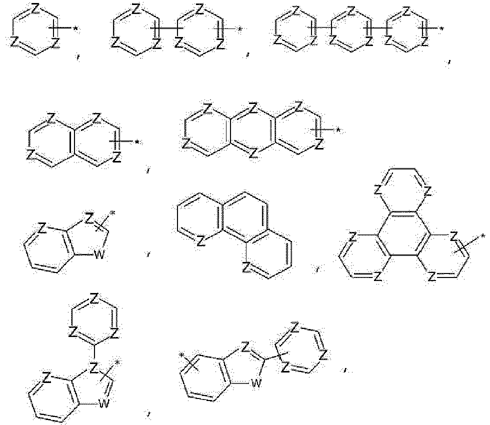 wherein, in Group 2,
W is each independently N, O, S, SO, SO₂, CR^{c}, CR^{d}R^{e}, SiR^{f} or SiR^{g}R^{h},
Z is each independently N, C or CR¹,
wherein R^{c} to Rⁱ are each independently hydrogen, deuterium, a substituted or unsubstituted C1 to C30 alkyl group, a substituted or unsubstituted C3 to C30 cycloalkyl group, a substituted or unsubstituted C3 to C30 heterocycloalkyl group, a substituted or unsubstituted C6 to C30 aryl group, a substituted or unsubstituted C3 to C30 heteroaryl group, a substituted or unsubstituted amine group, a substituted or unsubstituted C6 to C30 arylamine group, a substituted or unsubstituted C6 to C30 heteroarylamine group, a substituted or unsubstituted C1 to C30 alkoxy group, a substituted or unsubstituted C2 to C30 alkoxycarbonyl group, a substituted or unsubstituted C2 to C30 alkoxycarbonylamino group, a substituted or unsubstituted C7 to C30 aryloxycarbonylamino group, a substituted or unsubstituted C1 to C30 sulfamoylamino group, a substituted or unsubstituted C2 to C30 alkenyl group, a substituted or unsubstituted C2 to C30 alkynyl group, a substituted or unsubstituted silyl group, a substituted or unsubstituted silyloxy group, a substituted or unsubstituted C1 to C30 acyl group, a substituted or unsubstituted C1 to C20 acyloxy group, a substituted or unsubstituted C1 to C20 acylamino group, a substituted or unsubstituted C1 to C30 sulfonyl group, a substituted or unsubstituted C1 to C30 alkylthiol group, a substituted or unsubstituted C1 to C30 heterocyclothiol group, a substituted or unsubstituted C6 to C30 arylthiol group, a substituted or unsubstituted C1 to C30 heteroarylthiol group, a substituted or unsubstituted C1 to C30 ureide group, a halogen, a halogen-containing group, a cyano group, a hydroxyl group, an amino group, a nitro group, a carboxyl group, a ferrocenyl group, or a combination thereof, and
* is a linking point at which an element of the functional group is linked.

7. The organic compound of claim 6, wherein the substituted or unsubstituted functional group listed in Group 2 is a substituted or unsubstituted functional group listed in Group 3:

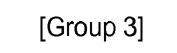

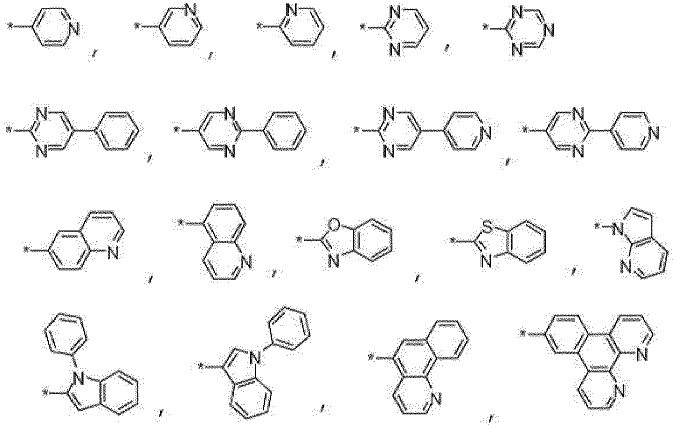

8. The organic compound of claim 1, which is compounds listed in Group 4:

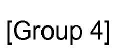

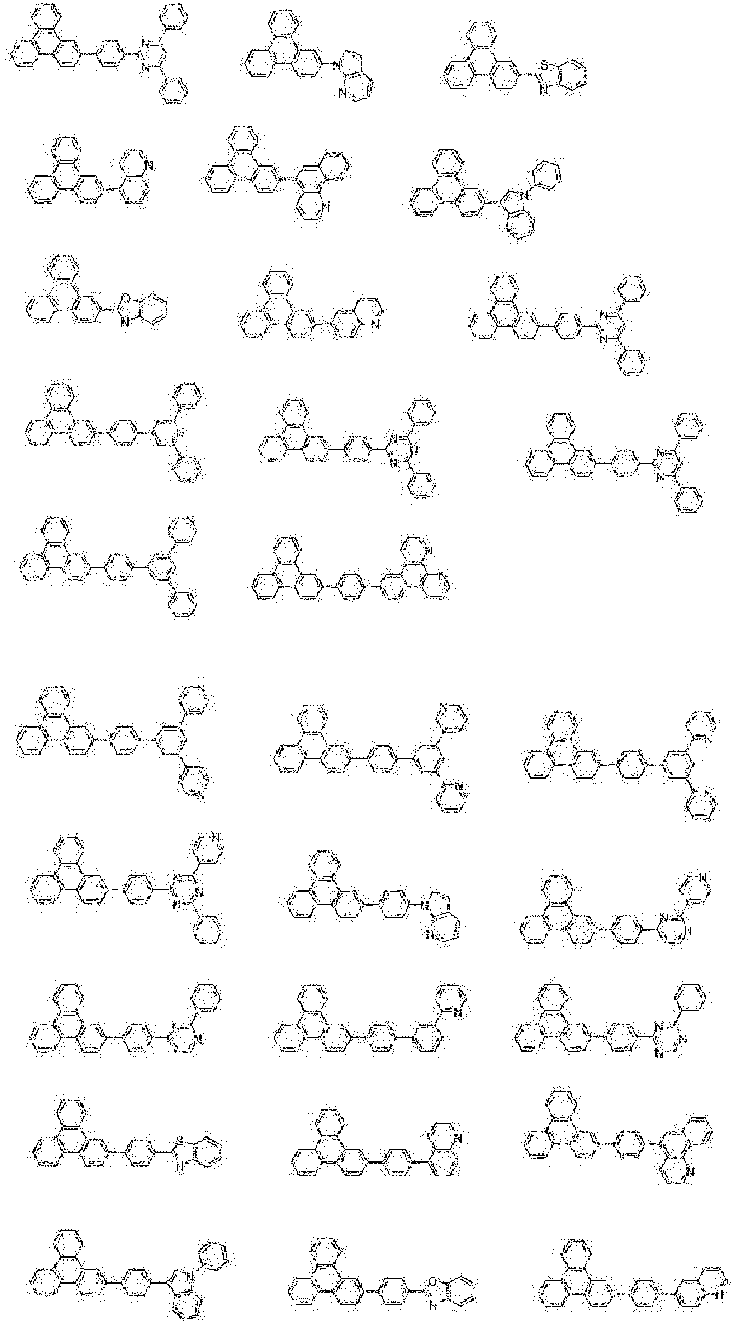

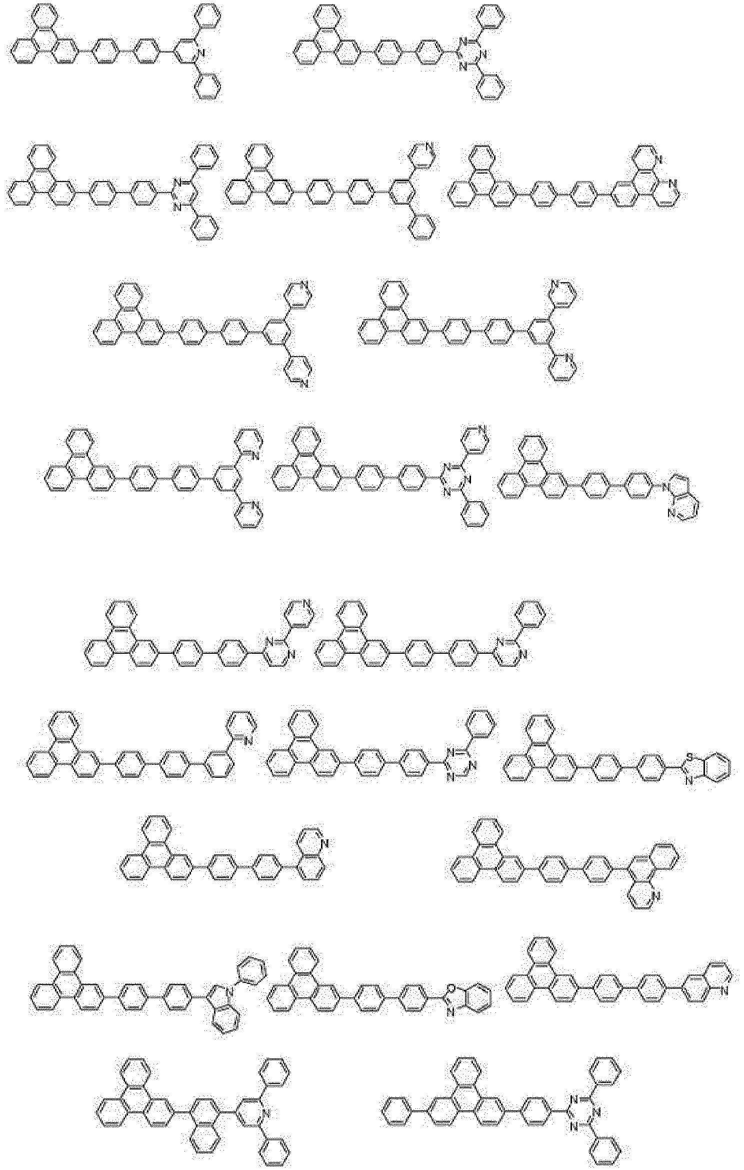

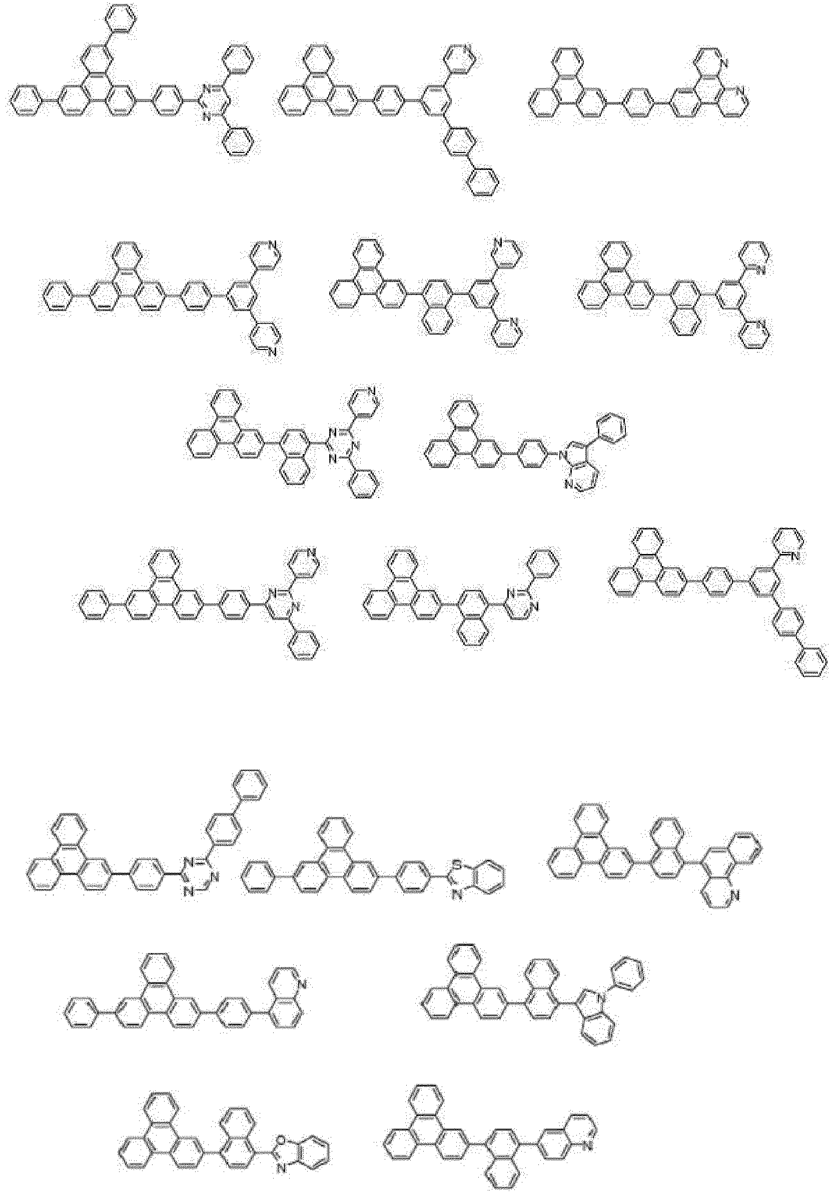

9. The organic compound of claim 1, wherein the organic compound has LUMO energy of -2.0 to -2.5eV.

10. An organic optoelectronic device comprising
an anode and a cathode facing each other,
at least one organic layer between the anode and the cathode,
wherein the organic layer comprises the organic compound of any one of claim 1 to claim 9.

11. The An organic optoelectronic device of claim 10, wherein
the organic layer comprises an emission layer,
the emission layer comprises the organic compound.

12. The organic optoelectronic device of claim 9, wherein the organic compound is included as a host in the emission layer.

13. The organic optoelectronic device of claim 10, wherein the organic layer comprises at least one auxiliary layer selected from a hole injection layer, a hole transport layer, an electron blocking layer, an electron transport layer, an electron injection layer, and a hole blocking layer, and
the auxiliary layer comprises the organic compound.

14. A display device comprising the organic optoelectronic device of claim 10.
